# EUROPÄISCHE PATENTSCHRIFT

(11) **EP 3 501 565 B1**
(45) Veröffentlichungstag und Bekanntmachung des Hinweises auf die Patenterteilung: **14.02.2024**
(21) Anmeldenummer: 18209392.2
(22) Anmeldetag: 30.11.2018
(51) Int. Cl.: A61M 1/16, A61L 2/18

(54) **BLUTBEHANDLUNGSMASCHINE MIT SOS-GENERATOR UND DESINFEKTIONSVERFAHREN**
BLOOD TREATMENT MACHINE WITH SOS GENERATOR AND DISINFECTION METHOD
MACHINE DE TRAITEMENT DU SANG POURVUE DE GÉNÉRATEUR SOS ET PROCÉDÉ DE DÉSINFECTION

(30) Priorität: 22.12.2017 DE 102017131104
(43) Veröffentlichungstag der Anmeldung: 26.06.2019
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB

(56) Entgegenhaltungen:
- DE-A1- 19 640 840
- DE-U1-202011 105 738
- US-A- 6 051 188

## Beschreibung

Bei der vorliegenden Erfindung handelt es sich um eine extrakorporale Blutbehandlungsmaschine mit einem integrierten SOS-Generator, sowie um eine verbesserte Methode zur Desinfektion von extrakorporalen Blutbehandlungsmaschinen.

### Hintergrund der Erfindung

In der chronischen Dialyse in einem Zentrum werden Dialysemaschinen von mehreren Patienten nacheinander benutzt. Daher muss vermieden werden, Keime von einem zum anderen Patienten zu übertragen. Heute wird deshalb in den meisten Fällen zwischen zwei Dialysebehandlungen eine Heißdesinfektion des Dialyseflüssigkeitskreises durchgeführt.

Je nach Gerätevariante und Hersteller liegt die Dauer des Gesamtprozesses dabei bei etwa 25 bis 40 Minuten. Ein solcher Prozess setzt sich im Wesentlichen aus drei Phasen zusammen:
- einer Aufheizphase, wobei eine Desinfektionsflüssigkeit, z.B. Wasser, aufgeheizt wird, bis etwa 85 °C erreicht sind, was im Allgemeinen ca. 15 min in Anspruch nimmt,
- einer Desinfektionsphase von ca. 15 min, während derer die verschiedenen zu desinfizierenden Flusswege des Dialyseflüssigkeitskreises mit der Desinfektionsflüssigkeit gespült werden und
- einer Phase, während derer das System ausgespült und abgekühlt wird, was erneut ca. 10 min in Anspruch nimmt.

Alternativ oder zusätzlich werden chemische kalte Desinfektionen des Dialyseflüssigkeitskreises und der Dialysemaschine durchgeführt. Dazu wird beispielsweise Hypochlorit oder Peressigsäure verwendet. Diese Art der Desinfektion wird beispielsweise nur wöchentlich durchgeführt.

Diese beiden Desinfektionsverfahren bringen einige Nachteile mit sich. So erfordert der heutige Heißdesinfektionsprozess beispielsweise einen hohen Zeit- und Energieaufwand.

Ferner werden bei der chemischen Desinfektion beispielsweise zusätzliche Chemikalien wie Zitronensäure, Hypochlorid oder Peressigsäure benötigt. Hierdurch entstehen zusätzliche Kosten und zudem müssen diese Chemikalien gehandhabt werden. Dies hat nicht nur einen erhöhten Aufwand zur Folge, sondern führt außerdem zu einer Vielzahl von potentiellen Fehlerquellen. Beispielsweise besteht dadurch, dass der Behälter mit Desinfektions- oder Entkalkungsmittel, beispielsweise Zitronensäure, meist an der Dialysemaschine angeschlossen bleibt, das Risiko, dass dieses Mittel während der Behandlung angesaugt werden könnte. Um dies zu verhindern, sind umfangreiche Schutzmaßnahmen auf Geräteseite erforderlich. Ferner ist beispielsweise Zitronensäure, wenn diese verschüttet wird, sehr klebrig und ätzend.

Ein weiterer Nachteil der chemischen Desinfektion besteht darin, dass die Leitfähigkeit mancher Mittel im Bereich der Leitfähigkeit von Konzentraten liegt, welche beim Aufbereiten von Dialyseflüssigkeit benötigt werden, sodass nicht sicher erkannt werden kann, ob statt dem entsprechenden Konzentrat ein Entkalkungsmittelkanister versehentlich angeschlossen würde.

Ferner sind die benötigten Chemikalien teilweise sehr aggressiv und greifen vor allem Edelstähle, bestimmte Kunststoffe und Keramiken an. Dies führt potentiell zu Korrosion von Systemkomponenten, weshalb höherwertige Materialien eingesetzt werden müssen.

Alternative Desinfektionsmittel, deren Einsatz beispielsweise in der Zahnmedizin bekannt ist, sind superoxidierte Lösungen (SOS: "super-oxidised solution" oder SOW: "super-oxidised water").

SOS sind Lösungen, welche durch elektrochemische Prozesse, insbesondere Elektrolyse, aus Wasser und Salzen (z.B. Natrium- oder Kaliumchlorid) hergestellt werden. Dabei werden beispielsweise Natriumhydroxid und Hypochlorsäure, sowie Chlor-Radikale erzeugt. Eine SOS als Desinfektionslösung zeigt sehr gute Desinfektionsergebnisse und greift einzellige Organismen an, insbesondere Pilze, Bakterien, Viren und Sporen, wohingegen mehrzellige Organismen, wie zum Beispiel menschliches Gewebe, nicht angegriffen werden.

Des Weiteren weisen SOS weitere Vorteile auf: Sie sind stabile Lösungen, nicht korrosiv und wirken im Allgemeinen schnell, können ph-neutral bis sauer und bis zu einem Jahr haltbar sein. Die Ausgangsstoffe für die Produktion von SOS sind günstig und das Endprodukt umweltfreundlich.

### Stand der Technik

Eine Heißdesinfektionsfunktion, wie sie vorstehend beschrieben wurde, ist beispielsweise in der Dialysemaschine 4008S classix von Fresenius Medical Care integriert. Ein weiteres Beispiel für ein Heißdesinfektionssystem ist Aquaboss von B. Braun, welches eine vollständige, totraumfreie Durchströmung des gesamten Dialyseflüssigkeitskreises oder Ringleitungssystems ermöglicht und in welchem ein Durchlauferhitzer heißes Wasser nur bei Bedarf produziert.

Während des Desinfektionsprozesses kann die Maschine nicht benutzt werden. Bestenfalls kann das Blutschlauchsystem für den nächsten Patienten aufgerüstet werden. Der Desinfektionsprozess ist somit ein bestimmender Faktor für die Zeitspanne zwischen 2 Behandlungen.

SOS, welche derzeit auf dem Markt kommerziell erhältlich sind, sind beispielsweise Sterilox, Microcyn oder EnviroNize Anolyte, für welche Generatoren, beispielsweise der EnviroLyte Generator von EnviroNize, auf dem Markt erhältlich sind.

Ferner ist aus DE 20 2011 105738 U1 eine Online-Desinfektion eines Dialysegerätes bekannt, welches es durch Integrierung einer Diamant-Elektrolyse-Zelle ermöglicht, In-Situ Oxidantien bzw. Desinfektionsmittel elektrolytisch zu generieren.

Aus DE 196 40 840 A1 ist eine Dialysemaschine mit einem NaOCl- Generator bekannt, welcher zur Desinfektion Natriumhypochlorit (NaOCl) durch Elektrolyse von NaCl erzeugt, wozu insbesondere im Dialysatkonzentrat vorhandenes NaCl verwendet wird.

Weiterer Stand der Technik ist in US 6 051 188 A offenbart, welcher ein Desinfektionsverfahren für eine Dialysemaschine mit Ozon aus einem Ozon-Generator und mit elektrolytisch in einem weiteren Generator hergestelltem NAOCl.

### Kurzbeschreibung der Erfindung

Folglich besteht eine Aufgabe der Erfindung darin, den Prozess der Desinfektion einer extrakorporalen Blutbehandlungsmaschinen, vorzugsweise einer Dialysemaschine, hinsichtlich der klinischen Abläufe und des Materialverbrauchs zu optimieren, sodass Nachteile, wie zum Beispiel die vorstehend beschriebenen, vermieden werden können. Ein Ziel ist es, die für einen Desinfektionsprozess erforderliche Zeit möglichst gering zu halten.

Diese Aufgabe wird erfindungsgemäß gelöst, indem ein SOS-Generator zur Herstellung von SOS als alternative Desinfektionslösung in eine extrakorporale Blutbehandlungsmaschine integriert wird, das heißt fester Bestandteil deren Hydraulik-/Flüssigkeitssystems ist. Erfindungsgemäß wurde erkannt, dass eine SOS auch basierend auf saurem Dialysekonzentrat hergestellt werden kann. Als der SOS-Generator kann ein kommerziell erhältlicher SOS-Generator, wie beispielsweise der vorstehend im Rahmen des Stands der Technik beschriebene SOS-Generator, sein.

Genauer wird die vorstehend beschriebene Aufgabe durch eine extrakorporale Blutbehandlungsmaschine, vorzugsweise eine Dialysemaschine, gelöst, in welche ein SOS-Generator integriert ist. Die extrakorporale Blutbehandlungsmaschine weist einen Flüssigkeitskreis (Ringleitung), vorzugsweise einen Dialyseflüssigkeitskreis, und eine Desinfektionsmittel-Zuführleitung auf, welche an den Flüssigkeitskreis angeschlossen und wahlweise entsperrbar ist, wodurch der SOS-Generator wahlweise an den Flüssigkeitskreis angeschlossen/fluidverbunden werden kann. Der SOS-Generator ist dafür angepasst, eine SOS zur Desinfektion des Flüssigkeitskreises aus Dialysekonzentrat und osmotischem Wasser herzustellen bzw. bereitzustellen, welches ggf. an der extrakorporalen Blutbehandlungsmaschine vorrätig/bevorratet ist.

Ferner wird bei der vorliegenden Erfindung bei Stillstandzeiten des Blutbehandlungssystems das Ablassen der SOS nach einer Desinfektion verhindert und verbleibt somit die SOS in dem Flüssigkeitskreis und ggf. in an diesen angeschlossenen Leitungen. Das Ablassen und Ausspülen (d.h. das Beenden des Desinfektionsprozesses) der SOS wird in diesem Fall vor der erneuten Inbetriebnahme der extrakorporalen Blutbehandlungsmaschine, beispielsweise vor einer ersten Behandlung am Folgetag, durchgeführt. Dies entspricht im Wesentlichen einer Verzögerung des Desinfektionsprozesses zwischen dem Einwirken und dem Ablassen der SOS um eine zusätzliche Zeitdauer, welche im Wesentlichen der Stillstandzeit entspricht. Alternativ oder zusätzlich ist der SOS-Generator dazu angepasst, den Flüssigkeitskreis, sowie vorzugsweise an den Flüssigkeitskreis angeschlossene Leitungen, unmittelbar vor Stillstandzeiten des Blutbehandlungssystems automatisch zu füllen.

Diese Anwendung ist dadurch möglich, dass SOS nicht korrosiv ist. Insbesondere bei längeren Stillstandzeiten der Blutbehandlungsmaschine, wie beispielsweise über Nacht, ist dieser Aspekt der vorliegenden Erfindung vorteilhaft. Wenn die SOS nach einem Desinfektionsprozess in dem Flüssigkeitskreis und ggf. den angeschlossenen Leitungen verbleibt, wird eine erneute Kontamination nahezu ausgeschlossen und die erneute Inbetriebnahme der extrakorporalen Blutbehandlungsmaschine wird beschleunigt. Alternativ oder zusätzlich kann die automatische Befüllung des Desinfektionsflüssigkeitskreises und ggf. der angeschlossenen Leitungen auch durchgeführt werden, ohne dass ein Desinfektionsprozess vorausgegangen ist, welcher Einwirken der SOS (z.B. durch Zirkulieren oder Spülen der SOS), Ablassen und Ausspülen der SOS in/aus dem Flüssigkeitskreis beinhaltet. In diesem Fall ist das während der Stillstandzeit stattfindende Keimwachstum stark reduziert. Dadurch ist auch vor der nächsten Verwendung, beispielsweise am Folgetag, ein unkomplizierter Desinfektionsprozess ausreichend.

Zuleitungen für Dialysekonzentrat und osmotisches Wasser zu dem SOS-Generator können an die gleiche Quelle oder Quellen für osmotisches Wasser und Elektrolyte bzw. Dialysekonzentrat angeschlossen sein, welche auch zur Bereitstellung frischer Dialyseflüssigkeit während einer extrakorporalen Blutbehandlung dienen.

Eine erfindungsgemäße Blutbehandlungsmaschine kann beispielsweise eine Hämodialyse-, Hämofiltrations- oder Hämodiafiltrationsmaschine sein. Im Falle eines offenen Flüssigkeitskreises sollte die Desinfektionsmittel-Zuführleitung vorteilhafterweise am Eingang des offenen Flüssigkeitskreises angeschlossen sein, wobei der Eingang beispielsweise ein Anschluss zum Zuführen frischer Dialyseflüssigkeit oder Komponenten davon zu dem Flüssigkeitskreis ist. In einem geschlossenen Flüssigkeitskreis kann der SOS-Generator durch die Desinfektionsmittel-Zuführleitung grundsätzlich an jeder Stelle des Flüssigkeitskreises angeordnet/angeschlossen werden, wobei ggf. zu beachten ist, dass einige Abschnitte oder Einheiten des Flüssigkeitskreises, wie der Dialysefilter/das Hohlfaserfiltermodul, kurzgeschlossen oder mit einer Bypass-Leitung umgangen werden können. Ebenfalls möglich ist es, dass der Flüssigkeitskreis, welcher während einer Dialysebehandlung ein offener Flüssigkeitskreis ist, während eines Desinfektionsprozesses kurzgeschlossen wird, indem dessen Eingang (z.B. der Anschluss zum Zuführen frischer Dialyseflüssigkeit) und Ausgang (z.B. ein Abfluss für verbrauchte Dialyseflüssigkeit) kurzgeschlossen werden, sodass die SOS in dem Flüssigkeitskreis zirkulieren kann. Im Rahmen eines Desinfektionsprozesses wird die SOS in den Flüssigkeitskreis (und ggf. Bypass-Leitungen, sowie ggf. andere, mit dem Flüssigkeitskreis fluidverbundene Leitungen) eingefüllt, einwirken gelassen und schließlich abgelassen und ausgespült. Das Einwirken kann stehendes Einwirken von SOS, Spülen mit SOS und/oder, insbesondere bei einem während des Desinfektionsprozesses geschlossenen Flüssigkeitskreis, Zirkulieren von SOS sowie Kombinationen daraus beinhalten und wird nachfolgend zusammengefasst als "Einwirken" bezeichnet. Die SOS wird vorzugsweise mit frischer Dialyseflüssigkeit oder Permeat aus der Ringleitung / dem Flüssigkeitskreis ausgespült. Ferner kann der SOS-Generator als Pumpe ausgebildet sein oder alternativ eine zusätzliche/separate Pumpe (z.B. eine Dosierpumpe) in der Desinfektionsmittel-Zuführleitung angeordnet sein, um die SOS in den Flüssigkeitskreis zu pumpen.

Saures Dialysekonzentrat, welches zur Herstellung von SOS geeignet ist, hat einen hohen Salzgehalt von mehr als 200 g/l NaCl. Dieses Dialysekonzentrat wird in einem Dialysezentrum in großen Mengen benötigt und befindet sich somit ohnehin an der Blutbehandlungsmaschine und/oder wird über eine zentrale Versorgung bereitgestellt. Dies gilt gleichermaßen für osmotisches Wasser. Somit kann bei der Desinfektion von extrakorporalen Blutbehandlungsmaschinen vorteilhafterweise auf teure und/oder aufwendig zu handhabende Desinfektionsmittel weitestgehend verzichtet werden und die laufenden Kosten, welche sowohl durch die Desinfektionsmittel an sich als auch durch aufwendige Sicherheitsmaßnahmen verursacht werden, deutlich reduziert werden. Dennoch ist es denkbar, Anschlüsse für derartige Desinfektionsmittel optional zusätzlich vorzusehen, sodass die Blutbehandlungsmaschine notfalls auch bei einem Defekt des SOS-Generators (fremd-) desinfiziert und darauf folgend zur Dialyse eingesetzt werden kann. Ebenso ist es denkbar, externe bzw. zusätzliche Anschlüsse für Wasser und Kochsalzlösung an dem SOS-Generator vorzusehen. Ferner kann das Dialyseflüssigkeitssystem / der Flüssigkeitskreis der erfindungsgemäßen Blutbehandlungsmaschine deutlich vereinfacht werden und der Energieaufwand (und ggf. Zeitaufwand) reduziert werden, da die Desinfektion bei Normaltemperatur stattfindet. Zudem wird die Haltbarkeit von weiteren Komponenten des Flüssigkeitskreises, insbesondere einem Dialyseflüssigkeitsfilter erhöht, da diese keiner Hitzebelastung unterliegen und durch die SOS nicht angegriffen werden. Dennoch ist es denkbar, SOS ggf. leicht vorzuwärmen, um eine Desinfektionsleistung der SOS noch zu steigern. Ein Desinfektionsprozess bei einer erfindungsgemäßen Blutbehandlungsmaschine ist leicht automatisierbar und schnell. Die Desinfektion kann innerhalb weniger Minuten durchgeführt werden, was insbesondere durch den Verzicht auf Aufheiz- und Abkühlvorgänge bedingt ist.

Allgemein betrachtet wird folglich durch den Einsatz eines in die Blutbehandlungsmaschine als fester Bestandteil integrierten SOS-Generators zur Desinfektion der Blutbehandlungsmaschine die Sicherheit des Systems sowie dessen Bedienung und Funktionalität deutlich erhöht und Kosten gesenkt.

Gemäß einem bevorzugten Aspekt der vorliegenden Erfindung hat der Flüssigkeitskreis einen Wasseraufbereitungsabschnitt, vorzugsweise einen Umkehrosmose-Abschnitt. Der SOS-Generator kann durch eine Wasser-Abführleitung oder Wasser-Entnahmeleitung stromabwärts des Wasseraufbereitungsabschnitts wahlweise an den Flüssigkeitskreis angeschlossen werden, in Bezug auf die Flussrichtung von Dialyseflüssigkeit oder Desinfektionsflüssigkeit in dem Flüssigkeitskreis.

In anderen Worten wird dem SOS-Generator das zur Erzeugung der SOS notwendige osmotische Wasser direkt von dem Wasseraufbereitungsabschnitt der Blutbehandlungsmaschine zugeführt. Ein solcher Wasseraufbereitungsabschnitt wird in vielen extrakorporalen Blutbehandlungsmaschinen zum Aufbereiten von Dialyseflüssigkeit eingesetzt und ist folglich ein in einer gängigen extrakorporalen Blutbehandlungsmaschine bereits vorhandener Abschnitt, welcher für die Desinfektion des Systems temporär umfunktioniert wird. Hierdurch kann auf zusätzliche Leitungen oder Behälter verzichtet werden. Es ist anzumerken, dass SOS nicht an Umkehrosmose-Filter eines Wasseraufbereitungsabschnitts gelangen darf und die Zirkulation von SOS in dem Flüssigkeitskreis sowie das Ausspülen nach der Desinfektion folglich nicht über diesen Wasseraufbereitungsabschnitt stattfinden darf. Ggf. ist eine Bypass-Leitung/Umgehungsleitung vorgesehen, um die SOS um den Wasseraufbereitungsabschnitt herum umzuleiten.

Gemäß einem weiteren bevorzugten Aspekt der vorliegenden Erfindung ist die Wasser-Abführleitung oder Wasser-Entnahmeleitung dazu vorgesehen, einen Teilstrom des osmotischen Wassers zu entnehmen. Dieser Teilstrom wird dem SOS-Generator zugeführt und dient zur Herstellung von SOS. Die vom SOS-Generator erzeugte SOS wird im Rahmen eines ausgewählten Desinfektionsprozesses in den Flüssigkeitskreis (vorzugsweise ohne zusätzliche Erwärmung) geleitet, gemäß einem weiteren bevorzugten Aspekt der vorliegenden Erfindung, durch eine an der Desinfektionsmittel-Zuführleitung angeordnete Dosierpumpe.

Ein solches Teilstromsystem ermöglicht es, den SOS-Generator sowie ggf. eine Dosierpumpe kleiner auszulegen bzw. zu dimensionieren, da beispielsweise nur ca. 10% des Gesamtbehandlungsvolumens benötigt werden, um für eine Desinfektion ausreichend SOS zu erzeugen. Als Dosierpumpe kann eine Verdrängerpumpe wie zum Beispiel eine Hubkolben-, Schlauch-, Membran- oder Zahnradpumpe eingesetzt werden.

Gemäß einem bevorzugten Aspekt der vorliegenden Erfindung ist ein Sensor stromabwärts des SOS-Generators in dem Flüssigkeitskreis vorgesehen, welcher dazu angepasst ist, außerhalb eines Desinfektionsprozesses die Chlorfreiheit zu überwachen und/oder mittels welchem während eines Desinfektionsprozesses die Zielkonzentration der SOS in dem Flüssigkeitskreis (durch eine Steuerungs-/Regelungseinheit) steuerbar ist. Zur Steuerung der Zielkonzentration kann eine Dosierpumpe gemäß einem der vorstehenden, bevorzugten Aspekte der Erfindung vorgesehen sein, es kann jedoch auch der SOS-Generator gesteuert werden, gezielt eine bestimmte Menge an SOS herzustellen.

Diese Sensoren können zueinander unterschiedliche sowie voneinander getrennte Sensoren sein, es kann jedoch auch ein einzelner Sensor verwendet werden, welcher für die Überwachung der Chlorfreiheit und/oder der Steuerung der SOS-Zielkonzentration genutzt wird. Für die Steuerung der Zielkonzentration ist es beispielsweise denkbar, den Sensor mit einer Dosierpumpe gemäß einem vorstehenden, bevorzugten Aspekt der Erfindung zu kombinieren oder den SOS-Generator selbst abhängig von den Sensordaten anzusteuern. Der Sensor bzw. die Sensoren sind vorzugsweise zur Messung von freiem Chlor, Gesamtchlor oder Chlordioxid geeignete Sensoren. Hierbei können bekannte Sensoren zum Einsatz kommen, wie beispielsweise Chloromax CC142D von Endress-Hauser. Die Sensoren können prinzipiell überall in dem Flüssigkeitskreis oder in mit diesem verbundenen und für SOS zugänglichen Leitungen angeordnet sein, vorteilhaft ist jedoch eine Anordnung in dem Flüssigkeitskreis direkt/unmittelbar nach dem Anschluss für die Desinfektionsmittel-Zuführleitung oder in der Desinfektionsmittel-Zuführleitung selbst. Wird der Sensor zur Überwachung der Chlorfreiheit zur Messung der SOS-Konzentration beim Ausspülen am Ende eines Desinfektionsprozesses eingesetzt, ist ein Einsatz an einem Abschnitt des Flüssigkeitskreises oder ggf. daran angeschlossenen Leitungen denkbar, in welchem eine Durchströmung ungleichmäßig ist bzw. ein Fluid gestaut werden könnte. Denkbar ist auch, den Sensor für die Überwachung der Chlorfreiheit in Leitungen, in welche keine SOS eindringen sollte, wie beispielsweise einem Anschluss des Wasseraufbereitungsabschnitts, einzusetzen.

Gemäß einem bevorzugten Aspekt der vorliegenden Erfindung ist vorzugsweise stromaufwärts von empfindlichen Einheiten, vorzugsweise des Wasseraufbereitungsabschnitts, eine Schutzeinrichtung zum Schutz dieser Einheiten vor Kontakt mit der SOS vorgesehen.

Diese Schutzeinrichtung ist dazu angepasst, SOS zu detektieren und eine Weiterleitung eines SOS enthaltenden Fluids zu unterbrechen. Hierfür ist beispielsweise an den empfindlichen Einheiten ein Sensor bereitgestellt, welcher die Anwesenheit von SOS an einer dieser empfindlichen Einheiten detektiert. Bei Anwesenheit von SOS (d.h. beispielsweise bei Überschreiten einer Konzentration von SOS) wird der Fluss des SOS-haltigen Fluids in Richtung der empfindlichen Einheiten unterbrochen, vorzugsweise indem eine Hardware-Sicherheitskette unterbrochen oder ein BUS-Signal erzeugt und an eine Controller-Einheit übertragen wird, welche ein Ventil, beispielsweise ein 3/2-Wege-Ventil, schaltet, um das SOS-haltige Fluid in einen Abfluss umzuleiten. Dabei kann beispielsweise einer der Sensoren zur Überwachung der Chlorfreiheit gemäß einem vorstehenden, bevorzugten Aspekt der Erfindung zum Detektieren von SOS zum Einsatz kommen. Vorteilhafterweise kann ein solches System zum Schutz von Umkehrosmose-Filtern des Wasseraufbereitungsabschnitts eingesetzt werden, welche nicht mit SOS in Kontakt kommen dürfen. Die Schutzeinrichtung ist dabei vorzugsweise stromaufwärts der empfindlichen Einheiten vorgesehen, kann jedoch beispielsweise auch zusätzlich stromabwärts davon angeordnet sein, um einen Rückstrom von SOS zu unterbinden. Alternativ können hierfür auch Ventile vorgesehen sein, welche einen Rückstrom unterbinden.

Gemäß einem bevorzugten Aspekt der vorliegenden Erfindung ist außenseitig an der extrakorporale Blutbehandlungsmaschine ein mit dem SOS-Generator verbundener Ablassanschluss, vorzugsweise ein Hahn, vorgesehen. Dieser ist für einen Nutzer zugänglich und dient dazu, dem Nutzer SOS als Desinfektionsmittel bereitzustellen.

Der Ablassanschluss (Zapfanschluss) kann hierbei manuell betätigbar sein und/oder automatisch öffnen und schließen, beispielsweise abhängig davon, ob etwas daran angeschlossen oder darunter gehalten wird. Das an diesem Ablassanschluss ablassbare SOS ist primär zur Oberflächendesinfektion vorgesehen. Dies können sowohl Oderflächen der Blutbehandlungsmaschine als auch von dieser unabhängige Oberflächen sein. Es sind jedoch auch andere Anwendungen denkbar wie beispielsweise zur Desinfektion von Patienten-Haut bevor ein Shunt gesetzt wird. Der Ablassanschluss kann beispielsweise mit einem Schlauch verbunden werden, um SOS gezielt an zu desinfizierende Flächen zu leiten. Alternativ kann der Ablassanschluss auch direkt zugänglich sein, um beispielsweise Desinfektionstücher mit SOS zu tränken. Vorteilhafterweise kann so auch SOS für Desinfektionen von Flüssigkeitskreisexternen oder sogar Blutbehandlungsmaschinenexternen Komponenten verwendet werden, wodurch die benötigte Menge anderer Desinfektionsmittel deutlich reduziert werden kann. Vorteilhafterweise kann der Ablassanschluss nicht nur während eines Desinfektionsprozesses genutzt werden, sondern auch während einer Dialysebehandlung, wofür der SOS-Generator beispielsweise von dem Flüssigkeitskreis abgeklemmt oder auf ein zuvor durch den SOS-Generator gefülltes Reservoir zurückgegriffen werden kann.

Gemäß einem bevorzugten Aspekt der vorliegenden Erfindung kann der Flüssigkeitskreis ein offener Kreis sein, wobei der SOS-Generator an Anschlüsse für Dialysekonzentrat und osmotisches Wasser angeschlossen ist.

Dies stellt die einfachste Variante des erfindungsgemäßen Systems dar und ist für existierende Blutbehandlungsmaschinen ggf. leicht nachrüstbar.

Ein weiterer, denkbarer (bevorzugter) Aspekt der vorliegenden Erfindung besteht darin, dass der Flüssigkeitskreis und/oder empfindliche Einheiten der Blutbehandlungsmaschine kurz geschlossen werden können, um SOS mittels einer Zirkulationspumpe innerhalb des Flüssigkeitskreises zu zirkulieren. Hierdurch kann wiederholt ein Teilstrom über den SOS-Generator zirkuliert werden, wodurch der Gehalt an SOS im Flüssigkeitskreis erhöht wird. Der SOS-Generator kann in diesem Fall kleiner ausgelegt werden. Allerdings werden die Desinfektionszeit und der apparative Aufwand hierdurch erhöht.

Denkbare Variationen und Ausführungen, welche bereits im Rahmen der Beschreibung der extrakorporalen Blutbehandlungsmaschine diskutiert wurden, sind auch auf entsprechende Elemente des nachfolgend beschriebenen Verfahrens anwendbar und werden nicht erneut beschrieben.

Die Aufgabe wird ferner gelöst durch ein Verfahren zur Desinfektion eines Flüssigkeitskreises (Ringleitung), vorzugsweise eines Dialyseflüssigkeitskreises, einer extrakorporalen Blutbehandlungsmaschine mit folgenden Schritten:
- Zuführen oder Bereitstellen/Bevorraten von Dialysekonzentrat und osmotischem Wasser zu/an einem in die extrakorporale Blutbehandlungsmaschine integrierten SOS-Generator;
- Erzeugen von superoxidierter Lösung oder SOS durch den SOS-Generator;
- Befüllen des Flüssigkeitskreises mit der SOS;
- Einwirken der SOS während einer vorermittelten Desinfektionsdauer; und
- Ablassen sowie Ausspülen der SOS nach Ablauf der Desinfektionsdauer bis eine Konzentration der im Flüssigkeitskreis vorhandenen (Rest-) SOS einen Konzentrationsgrenzwert unterschreitet.

Dabei kann das Einwirken sowohl stehendes Einwirken von SOS, Spülen mit SOS und/oder, insbesondere bei einem geschlossenen Flüssigkeitskreis, Zirkulieren von SOS als auch Kombinationen daraus beinhalten. Der anhand dieses Verfahrens durchführbare Desinfektionsprozess findet bevorzugt bei Raumtemperatur statt. Dadurch ist insbesondere keine Erwärmung der Desinfektionsflüssigkeit notwendig, wodurch folglich der Dialyseflüssigkeitskreis im Anschluss an eine Desinfektion auch nicht abgekühlt werden muss, um für eine Dialysebehandlung nutzbar zu sein. Dies führt zu deutlich kürzeren Prozesszeiten als bei einer Heißdesinfektion, insbesondere durch den Wegfall des Abkühlvorgangs. Dieses Verfahren kann sowohl manuell als auch automatisiert eingeleitet und durchgeführt werden. Ferner kann neben der Desinfektionsdauer optional auch eine benötigte Menge an SOS vorbestimmt werden, sodass der SOS-Generator gezielt nur die für einen Desinfektionsprozess benötigte Menge an SOS herstellt. Alternativ ist auch denkbar, dass der SOS-Generator die benötigte Menge an SOS für einen gewissen Behandlungszeitraum, beispielsweise einen Tag, vorproduziert und in einem Reservoir bereithält.

Ferner weist das Verfahren folgende Schritte auf:
- Ermitteln einer Stillstandzeit der Blutbehandlungseinrichtung; und
- Füllen des Flüssigkeitskreises sowie vorzugsweise an den Flüssigkeitskreis angeschlossene Leitungen mit SOS unmittelbar vor der Stillstandzeit und Ausführen eines Desinfektionsprozesses (mit Einwirken, Ablassen und Ausspülen der SOS) bzw. des erfindungsgemäßen Desinfektionsverfahrens vor der erneuten Inbetriebnahme der extrakorporalen Blutbehandlungsmaschine; oder
- Verhindern des Ablassens von SOS nach einer Desinfektion (d.h. nach dem Einwirken der SOS in dem Flüssigkeitskreis), unmittelbar vor der Stillstandzeit und Abschließen des Desinfektionsprozesses (Ablassen und Ausspülen der SOS) vor der erneuten Inbetriebnahme der extrakorporalen Blutbehandlungsmaschine.

Als erneute Inbetriebnahme der extrakorporalen Blutbehandlungsmaschine ist ein Ende der Stillstandzeit zu verstehen, also bevor eine neue Dialysebehandlung durchgeführt wird. Vorzugsweise erfolgt das Ausführen des Desinfektionsprozesses bzw. das Abschließen des Desinfektionsprozesses unmittelbar vor einer neuen Dialysebehandlung.

Gemäß einem bevorzugten Aspekt der vorliegenden Erfindung weist das Verfahren ferner einen Schritt auf, bei welchem der SOS-Generator über eine Wasser-Abführleitung mit Dialyseflüssigkeit zur Herstellung von SOS versorgt wird, welche an einem Wasseraufbereitungsabschnitt für verbrauchte Dialyseflüssigkeit (bzw. zum Bereitstellen von osmotischem Wasser) angeschlossen ist, der in den (nunmehr geschlossenen) Flüssigkeitskreis zwischengeschaltet (in Reihe geschaltet oder parallel geschaltet) ist.

Gemäß einem bevorzugten Aspekt der vorliegenden Erfindung weist das Verfahren ferner folgende Schritte auf:
- Abführen eines Stroms, vorzugsweise eines Teilstroms, von osmotischem Wasser aus dem Flüssigkeitskreis durch die Wasser-Abführleitung; und
- Zuführen der SOS von dem SOS-Generator in den Flüssigkeitskreis, vorzugsweise über eine Dosierpumpe.

Diese Schritte gemäß der beiden vorstehend beschriebenen, bevorzugten Aspekte werden bei einer Ausführungsform der Erfindung angewendet, in welcher der SOS-Generator das zur Herstellung von SOS benötigte osmotische Wasser direkt aus dem Flüssigkeitskreis bezieht.

Gemäß einem bevorzugten Aspekt der vorliegenden Erfindung weist das Verfahren ferner einen Schritt auf, bei welchem eine Zielkonzentration von SOS in dem Flüssigkeitskreis mit Hilfe eines Sensors durch eine Steuerungs-/Regelungseinheit gesteuert wird. Alternativ oder zusätzlich kann ein Sensor vorgesehen sein, welcher außerhalb eines Desinfektionsprozesses die Chlorfreiheit überwacht.

Die Steuerung der Zielkonzentration kann insbesondere dynamisch auf Basis von SOS-Konzentrationsdaten des Sensors erfolgen und mit Hilfe einer Dosierpumpe eingestellt werden. Alternativ kann der SOS-Generator dazu eingestellt sein, eine bestimmte Menge SOS zu produzieren. Ferner ist es auch möglich, die Zielkonzentration einmalig zu berechnen und anhand der Dosierpumpe oder der Bestimmung der SOS-Menge einzustellen, statt sie dynamisch zu steuern.

Gemäß einem bevorzugten Aspekt der vorliegenden Erfindung weist das Verfahren ferner folgende Schritte auf:
- Erkennen von SOS durch einen Sensor einer Schutzeinrichtung, welche (vorzugsweise stromaufwärts) unmittelbar neben empfindlichen Einheiten, vorzugsweise einem Wasseraufbereitungsabschnitt, in dem Flüssigkeitskreis angeordnet ist; und
- Aktivieren der Schutzeinrichtung, welche ein Weiterleiten der SOS zu den empfindlichen Einheiten verhindert, insbesondere ein Ablassen der SOS initiiert.

### Figurenbeschreibung

Die Erfindung wird im Folgenden anhand bevorzugter Ausführungsformen beschrieben, wobei Merkmale verschiedener Ausführungsformen untereinander ausgetauscht und/oder kombiniert werden können. Es versteht sich, dass Einzelheiten der beschriebenen bevorzugten Ausführungsformen die Erfindung als solche nicht beschränken und sich für den Fachmann naheliegend verschiedenartige Änderungen, Modifikationen und/oder Äquivalente ergeben können, die als solche allesamt im Rahmen des durch die beigefügten Ansprüche definierten Schutzbereichs der Erfindung liegen. Ferner werden in der nachfolgenden Beschreibung der bevorzugten Ausführungsformen gleiche Bezugszeichen für einander entsprechende Merkmale verwendet, welche ggf. nur einmal beschrieben werden, jedoch in jeder der vorgestellten Ausführungsformen vorgesehen sein können.
Fig. 1 zeigt eine Prinzipskizze einer Ausführungsform mit offenem Flüssigkeitskreis;
Fig. 2 zeigt eine Prinzipskizze einer zweiten Ausführungsform mit geschlossenem Flüssigkeitskreis;
Fig. 3 zeigt ein Flussdiagramm für ein Beispiel des erfindungsgemäßen Verfahrens,
Fig. 4 zeigt ein Flussdiagramm für ein erfindungsgemäßes Teilverfahren für eine Ausführungsform der Erfindung, bei welcher der SOS-Generator osmotisches Wasser aus dem Flüssigkeitskreis bezieht;
Fig. 5 zeigt ein Flussdiagramm für ein erfindungsgemäßes Teilverfahren für einen bevorzugten Aspekt der Ausführungsform gemäß Fig. 4;
Fig. 6 zeigt ein Flussdiagramm für ein erfindungsgemäßes Teilverfahren für eine Ausführungsform der Erfindung, wobei eine Dosierpumpe zur Zufuhr von SOS zu dem Flüssigkeitskreis vorgesehen ist;
Fig. 7 zeigt ein Flussdiagramm für ein erfindungsgemäßes Teilverfahren für eine Ausführungsform der Erfindung, wobei eine Schutzeinrichtung für den Schutz bestimmter Einheiten vor Kontakt mit SOS vorgesehen ist; und
Fig. 8a und 8b zeigen Flussdiagramme für erfindungsgemäße Teilverfahren, welche gewährleisten, dass während einer Stillstandzeit der Flüssigkeitskreis mit SOS befüllt ist.

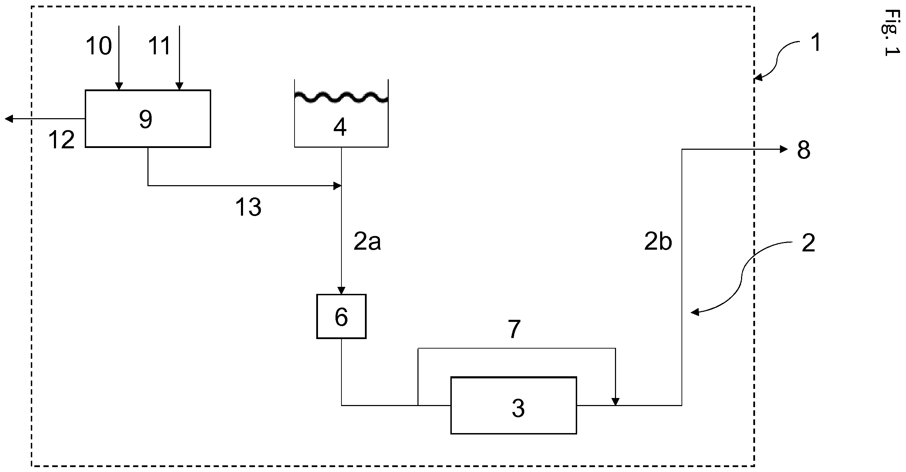

Fig. 1 zeigt eine Prinzipskizze von einer einfachen Ausführungsform einer Dialyseflüssigkeitsseite einer erfindungsgemäßen extrakorporalen Blutbehandlungsmaschine 1. Eine Blutseite der extrakorporalen Blutbehandlungsmaschine 1 ist nicht abgebildet, um die Darstellung zu vereinfachen. Zu dem gleichen Zweck ist ferner die Darstellung der Dialyseflüssigkeitsseite bzw. des Flüssigkeitskreises 2 reduziert, sodass lediglich für die Erläuterung der Erfindung notwendige Elemente eines Flüssigkeitskreises 2 dargestellt sind. Es versteht sich, dass neben diesen dargestellten Elementen die extrakorporale Blutbehandlungsmaschine einen Blutkreis aufweist, welcher während einer Dialysebehandlung über einen Shunt an Gefäße eines Patienten anschließbar ist und diverse Komponenten wie zum Beispiel eine Blutpumpe, Druck- und Temperatursensoren und eine Luftfalle aufweisen kann, wobei durch den Flüssigkeitskreis 2 geleitete Dialyseflüssigkeit und durch den Blutkreis geleitetes Patientenblut im Rahmen einer Dialysebehandlung zum Austausch von urämischen Toxinen sowie ggf. von Elektrolyten und Wasser zwischen Blut und Dialyseflüssigkeit im Gegenstromprinzip durch ein Hohlfaserfiltermodul 3 geleitet werden. Ferner versteht es sich, dass auch ein Flüssigkeitskreis 2 weitere Elemente wie Elektrolytzuleitungen, Mischer, Pumpen, Sensoren und Ventile aufweist. Diese nicht dargestellten Elemente sind aus dem Stand der Technik bekannt und werden nachfolgend nicht genauer beschrieben. Dies gilt gleichermaßen für alle nachfolgend beschriebenen Ausführungsformen der Erfindung.

Die Ausdrücke "stromabwärts" und "stromaufwärts" beziehen sich stets auf eine Flussrichtung von Dialysemittel in dem Flüssigkeitskreis 2 während einer Dialysebehandlung bzw. auf eine Flussrichtung von Desinfektionsmittel in dem Flüssigkeitskreis 2 während eines Desinfektionsprozesses.

Die in Fig. 1 dargestellte extrakorporale Blutbehandlungsmaschine 1 hat einen offenen Flüssigkeitskreis/Ringleitung 2, vorzugsweise einen Dialyseflüssigkeitskreis. Dieser hat einen Anschluss an zumindest eine Quelle (Reservoir/Zuleitung) 4, hier vereinfacht als Flüssigkeitsbehälter dargestellt, zur Bereitstellung frischer Dialyseflüssigkeit bzw. Komponenten davon, wie osmotischem Wasser, welches beim Fließen durch einen Zuleitungsabschnitt 2a des Flüssigkeitskreises 2, vorzugsweise durch in dem Zuleitungsabschnitt 2a angeordneten Mischkammern, mit Elektrolyten zu frischer Dialyseflüssigkeit vermischt werden. Die zumindest eine Quelle 4 kann beispielsweise ein an/in der Blutbehandlungsmaschine vorgesehenes Reservoir oder ein Anschluss an eine Blutbehandlungsmaschinen-externe Dialyseflüssigkeitsquelle sein.

Die Quelle für frische Dialyseflüssigkeit 4 ist an den Zuleitungsabschnitt 2a des Flüssigkeitskreises 2 angeschlossen (mit diesem fluidverbunden). Der Zuleitungsabschnitt 2a weist vorzugsweise unmittelbar stromabwärts von der Anschlussstelle, an welcher der Zuleitungsabschnitt 2a mit der zumindest einen Quelle 4 fluidverbunden ist, einen Anschluss für eine Desinfektionsmittel-Zuführleitung 13 auf. Vorzugsweise unmittelbar stromabwärts von der Anschlussstelle, an welcher die Desinfektionsmittel-Zuführleitung 13 an den Zuleitungsabschnitt 2a anschließbar oder angeschlossen ist, ist ein Sensor 6 in dem Zuleitungsabschnitt 2a angeordnet, welcher zur Messung von SOS, insbesondere von freiem Chlor, Gesamtchlor oder Chlordioxid, geeignet ist und ggf. zur Überwachung der Chlorfreiheit (z.B. beim Ausspülen der SOS) und/oder zur Einstellung einer SOS-Zielkonzentration in dem Flüssigkeitskreis 2 dient.

Der Zuleitungsabschnitt 2a ist ferner stromabwärts des Sensors 6 mit einem Hohlfaserfiltermodul 3 fluidverbunden. In diesem Hohlfaserfiltermodul 3 findet während einer Dialysebehandlung der Austausch zwischen Dialyseflüssigkeit und Blut statt und während des Desinfektionsprozesses kann es zur Desinfektion des Flüssigkeitskreises 2 mit Hilfe einer Bypass-Leitung 7 kurzgeschlossen sein. Die Bypass-Leitung 7 ist während eines Desinfektionsprozesses unmittelbar stromaufwärts des Hohlfaserfiltermoduls 3 mit dem Zuleitungsabschnitt 2a fluidverbunden und unmittelbar stromabwärts des Hohlfaserfiltermoduls 3 mit einem Ableitungsabschnitt 2b des Flüssigkeitskreises 2 fluidverbunden, wobei der Durchfluss durch das Hohlfaserfiltermodul 3 gesperrt ist, sodass die SOS um das Hohlfasermodul 3 herum umgeleitet wird. Die Sperrung des Durchflusses durch das Hohlfasermodul 3 (Trennung der Fluidverbindung zwischen Hohlfasermodul 3 und Flüssigkeitskreis 2) kann beispielsweise durch Sperrventile oder durch Abnehmen des vorzugsweise als Einwegteil ausgebildeten Hohlfaserfiltermoduls 3 realisiert sein.

Der Ableitungsabschnitt 2b ist dazu angepasst, während einer Dialysebehandlung verbrauchte Dialyseflüssigkeit von dem Hohlfaserfiltermodul 3 abzuleiten und mündet in einen Abfluss 8 zur Entsorgung von verbrauchter Dialyseflüssigkeit, welche auf diesem Weg (über den Ableitungsabschnitt 2b und den Abfluss 8) entsorgt werden kann. Der Abfluss kann zusätzlich an einen Sammelbehälter (nicht dargestellt) zum Sammeln von verbrauchter Dialyseflüssigkeit angeschlossen sein. Ferner ist der Ableitungsabschnitt 2b und der Abfluss 8 dazu konfiguriert, während eines Desinfektionsprozesses SOS abzuleiten.

Ferner ist ein SOS-Generator 9 in die extrakorporale Blutbehandlungsmaschine 1 integriert (ein fester Bestandteil einer Blutbehandlungsmaschinen-internen Hydraulik). Dieser ist mit Zuleitungen für Dialysekonzentrat 10 und osmotisches Wasser 11 fluidverbunden, über welche dem SOS-Generator 9 notwendige Komponenten für die Herstellung von SOS zugeführt werden.

Der SOS-Generator 9 weist einen Ablassanschluss (Zapfanschluss) 12 auf, welcher eine Fluidverbindung zu dem die Blutbehandlungsmaschine 1 umgebenden Raum ermöglicht und welcher SOS auch zur Nutzung außerhalb der Blutbehandlungsmaschine 1 bereitstellt.

Der SOS-Generator 9 ist mit einer Desinfektionsmittel-Zuführleitung 13 fluidverbunden, welche dazu angepasst ist, von dem SOS-Generator 9 hergestellte SOS dem Flüssigkeitskreis 2 zuzuführen. Hierfür ist der SOS-Generator 9 wahlweise mit einer Pumpe ausgestattet oder alternativ ist eine zusätzliche (separate) Pumpe in der Desinfektionsmittel-Zuführleitung 13 angeordnet. Somit ist die Desinfektionsmittel-Zuführleitung 13 wahlweise anschließbar (fluidverbindbar) an einen Eingang des offenen Flüssigkeitskreises 2, d.h. unmittelbar stromabwärts von der Anschlussstelle, an welcher der Zuleitungsabschnitt 2a mit der zumindest einen Quelle 4 fluidverbunden ist.

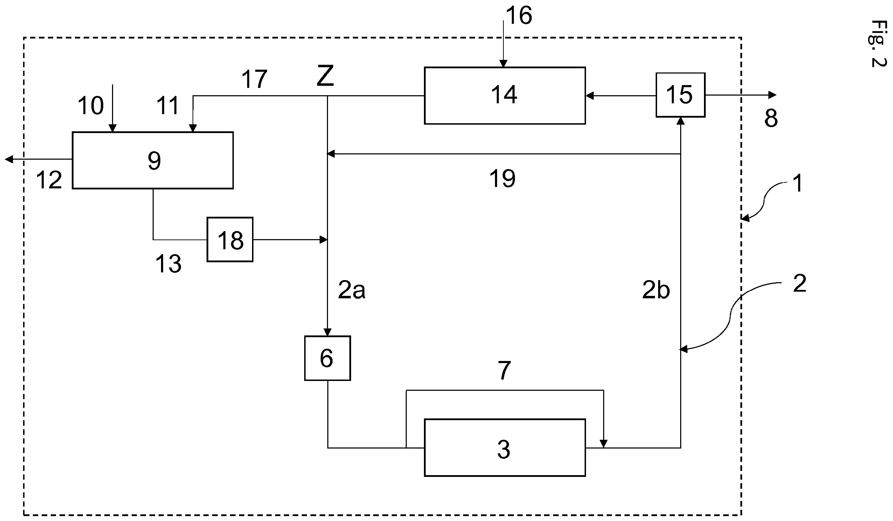

Fig. 2 zeigt eine zweite bevorzugte Ausführungsform der erfindungsgemäßen extrakorporalen Blutbehandlungsmaschine, welche auf der vorstehend anhand von Fig. 1 beschriebenen Ausführungsform aufbaut. Dementsprechend werden einander entsprechende Elemente mit gleichen Referenzzeichen referenziert und werden nicht erneut beschrieben.

Anders als in der in Fig. 1 dargestellten Blutbehandlungsmaschine ist in der Ausführungsform nach Fig. 2 ein geschlossener Flüssigkeitskreis 2 mit einem Wasseraufbereitungsabschnitt 14 vorgesehen, welcher dazu angepasst ist, durch Umkehrosmose osmotisches Wasser bereitzustellen/herzustellen.

Während einer Dialysebehandlung wird von dem Hohlfaserfiltermodul 3 verbrauchte Dialyseflüssigkeit in dem Ableitungsabschnitt 2b des Flüssigkeitskreises 2 geleitet. Der Ableitungsabschnitt 2b ist mit einem Eingang des Wasseraufbereitungsabschnitts 14 fluidverbunden oder verbindbar und unmittelbar stromaufwärts von dem Wasseraufbereitungsabschnitt 14 ist in dem Ableitungsabschnitt 2b eine Schutzeinrichtung 15 angeordnet. Diese Schutzeinrichtung 15 dient zum Schutz des Wasseraufbereitungsabschnitts 14, insbesondere von in diesem enthaltenen Umkehrosmose-Filtern, insbesondere während eines Desinfektionsprozesses, und wird später genauer beschrieben. Die im Rahmen einer Dialysebehandlung von dem Hohlfaserfiltermodul 3 abgeleitete, verbrauchte Dialyseflüssigkeit wird also in den Ableitungsabschnitt 2b, über die Schutzeinrichtung 15, zumindest teilweise in den Wasseraufbereitungsabschnitt 14 geleitet, wo sie durch Umkehrosmose aufbereitet wird und zur Herstellung von osmotischem Wasser dient. Der Wasseraufbereitungsabschnitt 14 weist zusätzlich eine Wasser-Zuführleitung 16 auf, welche dazu angepasst ist, dem Wasseraufbereitungsabschnitt 14 zusätzlich oder alternativ zu der verbrauchten Dialyseflüssigkeit Wasser zur Herstellung von osmotischem Wasser zuzuführen/bereitzustellen.

Der Ausgang des Wasseraufbereitungsabschnitts 14 zweigt an einer Zweigstelle Z in den Zuleitungsabschnitt 2a des Flüssigkeitskreises 2 ab, der zum Eingang des Hohlfaserfiltermoduls 3 führt. Der Ausgang des Wasseraufbereitungsabschnitts 14 zweigt an der Zweigstelle Z ferner in eine Wasser-Abführleitung 17 ab, welche in den SOS-Generator 9 mündet, der wiederum über die Desinfektionsmittel-Zuführleitung 13 zum Durchführen eines Desinfektionsprozesses wahlweise mit dem Zuleitungsabschnitt 2a stromab zur Zweigstelle Z fluidverbindbar ist. In dem Zuleitungsabschnitt 2a ist unmittelbar nach dem Anschluss für die Desinfektionsmittel-Zuführleitung 13 der Sensor 6 zur Messung von einer SOS-Konzentration vorgesehen. Ferner ist in dem Zuleitungsabschnitt 2a ein Mischabschnitt (nicht gesondert dargestellt) vorgesehen, welcher dazu angepasst ist, während einer Dialysebehandlung das von dem Wasseraufbereitungsabschnitt 14 bereitgestellte osmotische Wasser mit Elektrolyten bzw. Dialysekonzentrat zu mischen und so frische Dialyseflüssigkeit bereitzustellen. Ferner ist der Zuleitungsabschnitt 2a dazu angepasst, die frische Dialyseflüssigkeit dem Hohlfaserfiltermodul 3 zuzuführen.

Während eines Desinfektionsprozesses ist die Wasser-Abführleitung 17 mit dem Zuleitungsabschnitt 2a an der Zweigstelle Z unmittelbar stromabwärts des Wasseraufbereitungsabschnitts 14 fluidverbunden. Die Wasser-Abführleitung 17 ist ferner mit dem SOS-Generator 9 fluidverbunden und dazu angepasst, einen Teilstrom von durch den Wasseraufbereitungsabschnitt 14 bereitgestelltem, osmotischem Wasser dem SOS-Generator 9 zuzuführen, während das restliche osmotische Wasser in den Zuleitungsabschnitt 2a abzweigt. Der SOS-Generator 9 erzeugt aus dem durch die Wasser-Abführleitung 17 bereitgestellten osmotischen Wasser und durch eine Zuleitung für Dialysekonzentrat 10 bereitgestelltem Dialysekonzentrat bzw. Elektrolyten SOS. Der SOS-Generator 9 ist mit der Desinfektionsmittel-Zuführleitung 13 fluidverbunden. In der Desinfektionsmittel-Zuführleitung 13 ist eine Dosierpumpe 18 vorgesehen, welche eine dosierte Menge SOS in den während des Desinfektionsprozesses mit der Desinfektionsmittel-Zuführleitung 13 fluidverbundenen Zuleitungsabschnitt 2a pumpt, wo die SOS sich als Teilstrom mit dem in dem Zuleitungsabschnitt 2a fließenden osmotischen Wasser vermischt. Der Sensor 6 misst eine SOS-Konzentration in dem Zuleitungsabschnitt 2a und gibt ein Konzentrationssensorsignal aus, auf dessen Basis die Dosierpumpe 18 steuerbar ist, um eine Zielkonzentration von SOS in dem Zuleitungsabschnitt 2a einzustellen.

Denkbar ist prinzipiell auch, alternativ die Fluidverbindung zwischen dem Anschluss für die Wasser-Abführleitung 17 und dem Anschluss für die Desinfektionsmittel-Zuführleitung 13 zu unterbrechen und anstelle eines Teilstroms den Gesamtstrom über den SOS-Generator 9 zu zirkulieren, wobei eine Dosierpumpe 18 nicht notwendig ist und die Konzentration der SOS durch den SOS-Generator 9 direkt eingestellt werden kann.

In der in Fig. 2 dargestellten Ausführungsform ist während eines Desinfektionsprozesses die Bypass-Leitung 7 vorgesehen, welche eine erste Bypass-Leitung ist und welche wahlweise an den Zuleitungsabschnitt 2a und an den Ableitungsabschnitt 2b anschließbar (fluidverbindbar) ist, um SOS um das Hohlfaserfiltermodul 3 herumzuleiten und dieses aus dem Flüssigkeitskreis 2 auszukoppeln. Ferner ist eine zweite Bypass-Leitung 19 unmittelbar vor der Schutzeinrichtung 15 mit dem Ableitungsabschnitt 2b fluidverbindbar bzw. während eines Desinfektionsprozesses mit dem Ableitungsabschnitt 2b und mit dem Zuleitungsabschnitt 2a, vorzugsweise stromaufwärts des Mischabschnitts, fluidverbunden. Die zweite Bypass-Leitung 19 ist somit dazu angepasst, die SOS von dem Ableitungsabschnitt 2b in den Zuleitungsabschnitt 2a zurückzuführen und dabei die SOS um den Wasseraufbereitungsabschnitt 14 und die Schutzeinrichtung 15 herumzuleiten. Auf diese Weise ist die SOS in dem Flüssigkeitskreis 2 zirkulierbar.

Der Schutzabschnitt 15, welcher unmittelbar stromabwärts der Stelle angeordnet ist, an welcher die zweite Bypass-Leitung 19 mit dem Ableitungsabschnitt 2b verbunden ist, weist einen Sensor auf, welcher eine SOS-Konzentration misst, wobei bei Überschreiten eines Konzentrationsgrenzwertes der Schutzabschnitt 15 den Flüssigkeitskreis 2 unterbricht, insbesondere SOS-haltiges Fluid zu einem Abfluss 8 umleitet, sodass kein SOS-haltiges Fluid zu dem Wasseraufbereitungsabschnitt 14 weitergeleitet werden kann.

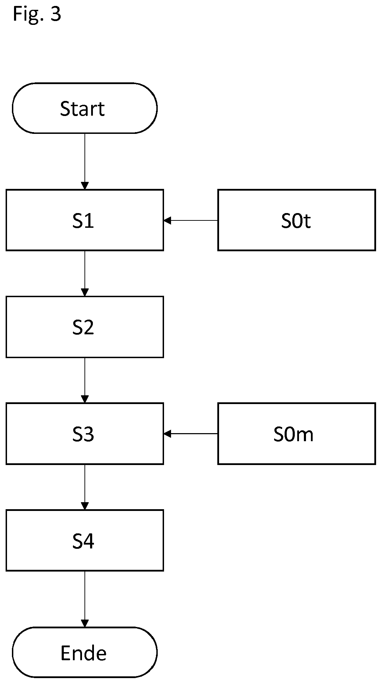

Fig. 3 zeigt ein Flussdiagramm für ein Beispiel eines erfindungsgemäßen Verfahrens, welcher ein Desinfektionsprozess ist. Dabei werden die benötigte Desinfektionszeit S0t und ggf. die benötigte Menge an SOS S0m vorermittelt. In einem ersten Schritt S1 wird eine ggf. vorermittelte Menge SOS erzeugt. Anschließend wird in einem zweiten Schritt S2 der Desinfektionsflüssigkeitskreis und ggf. die daran angeschlossenen Leitungen mit SOS befüllt. In einem dritten Schritt S3 wird die SOS während der vorbestimmten Desinfektionszeit einwirken gelassen. In einem vierten Schritt S4 wird die SOS abgelassen und ausgespült, bis eine Konzentration der SOS, welche durch einen Sensor ermittelt wird, einen vorbestimmten Grenzwert unterschreitet.

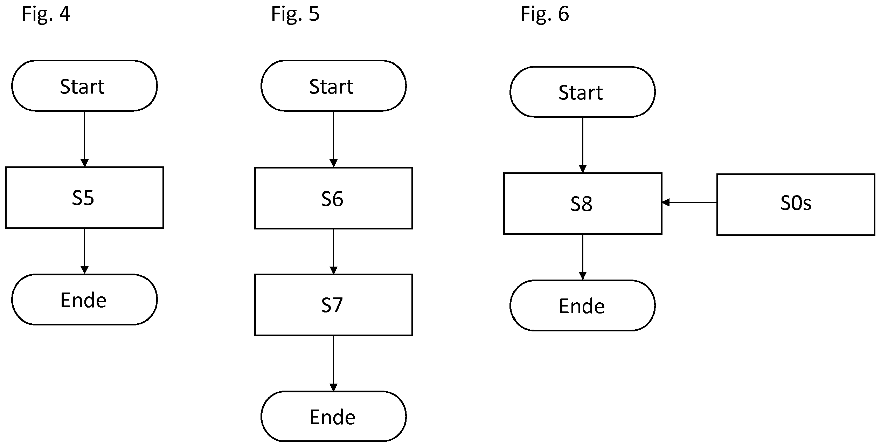

Fig. 4 zeigt ein Flussdiagramm für einen Schritt S5 eines erfindungsgemäßen Teilverfahrens, für eine Ausführungsform, bei welcher der SOS-Generator osmotisches Wasser aus dem Flüssigkeitskreis 2 bezieht. Dabei wird in Schritt S5 der SOS-Generator 9 an den Flüssigkeitskreis 2 durch eine Wasser-Abführleitung 17 an den Flüssigkeitskreis 2 angeschlossen.

Fig. 5 zeigt ein Flussdiagramm für Schritte eines erfindungsgemäßen Teilverfahrens, wobei in Schritt S6 ein Teilstrom von osmotischem Wasser aus dem Flüssigkeitskreis 2 durch die Wasser-Abführleitung 17 an den SOS-Generator 9 geleitet wird und in Schritt S7 die von dem SOS-Generator 9 erzeugte SOS, vorzugsweise durch eine Dosierpumpe 18, in den Flüssigkeitskreis 2 geleitet wird.

Fig. 6 zeigt ein Flussdiagramm für Schritte eines erfindungsgemäßen Teilverfahrens, wobei in Schritt S0s anhand eines Sensors eine SOS-Konzentration in dem Flüssigkeitskreis 2 gemessen wird und wobei in Schritt S8 eine Zielkonzentration von SOS in dem Flüssigkeitskreis 2, vorzugsweise durch eine Dosierpumpe 18, eingestellt wird. Dieses Teilverfahren ist insbesondere dann einsetzbar, wenn die SOS mittels eines Teilstromprinzips erzeugt wird, bei welchem ein Teilstrom aus dem Flüssigkeitskreis 2 abgeführt und dem SOS-Generator zugeführt wird und daraus hergestellte SOS wieder dem Flüssigkeitskreis 2, vorzugsweise mit Hilfe einer Dosierpumpe 18, zugeführt wird.

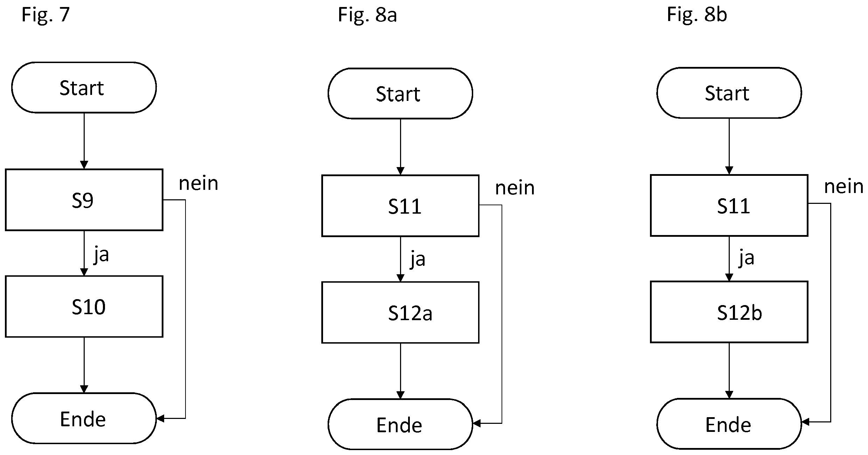

Fig. 7 zeigt ein Flussdiagramm für Schritte eines erfindungsgemäßen Teilverfahrens, wobei in Schritt S9 durch einen Sensor einer Schutzeinrichtung 15 eine Anwesenheit von SOS stromaufwärts von empfindlichen Einheiten, wie einem Wasseraufbereitungsabschnitt 14, erkannt wird und in Schritt S10 bei Erkennen einer Anwesenheit von SOS die Schutzeinrichtung 15 aktiviert wird, um das SOS-haltige Fluid umzuleiten bzw. ein Weiterfließen zu den empfindlichen Einheiten zu unterbinden.

Fig. 8a und Fig. 8b zeigen Flussdiagramme für Schritte von erfindungsgemäßen Teilverfahren, welche gewährleisten, dass während einer Stillstandzeit der Flüssigkeitskreis 2 mit SOS befüllt ist. Dabei wird in Schritt S11 jeweils ermittelt, ob eine Stillstandzeit der extrakorporalen Blutbehandlungsmaschine bevorsteht. In dem Teilverfahren gemäß Fig. 8a, in Schritt S12a, wird bei Ermittlung einer Stillstandzeit der Flüssigkeitskreis 2 sowie daran angeschlossene Leitungen unmittelbar bevor der Stillstandzeit und/oder nach der letzten Dialysebehandlung mit SOS befüllt. Dieses Teilverfahren kann auch stattfinden, bevor ein Desinfektionsprozess stattgefunden hat, wobei ein Desinfektionsprozess vor der nächsten Verwendung, beispielsweise am Folgetag, stattfinden kann. In dem Teilverfahren gemäß Fig. 8b, in Schritt S12b, dagegen wird bei Ermittlung einer Stillstandzeit in Anschluss an eine Desinfektion das Ablassen und Ausspülen der SOS verhindert und der Desinfektionsprozess kann vor der nächsten Verwendung, beispielsweise am Folgetag beendet werden.

### Referenzzeichenliste

- 1: Extrakorporale Blutbehandlungsmaschine
- 2: Flüssigkeitskreis/Ringleitung
- 3: Hohlfaserfiltermodul
- 4: Zumindest eine Quelle für Dialyseflüssigkeit oder Komponenten davon
- 6: Sensor
- 7: (Erste) Bypass-Leitung
- 8: Abfluss
- 9: SOS-Generator
- 10: Zuleitungen für Dialysekonzentrat
- 11: Zuleitungen für osmotisches Wasser
- 12: Ablassanschluss / Zapfanschluss
- 13: Desinfektionsmittel-Zuführleitung
- 14: Wasseraufbereitungsabschnitt
- 15: Schutzeinrichtung
- 16: Wasser-Zuführleitung
- 17: Wasser-Abführleitung / Wasser-Entnahmeleitung
- 18: Dosierpumpe
- 19: Zweite Bypass-Leitung
- Z: Zweigstelle

## Patentansprüche

1. Extrakorporale Blutbehandlungsmaschine, vorzugsweise Dialysemaschine, mit
einem Flüssigkeitskreis (2), vorzugsweise einen Dialyseflüssigkeitskreis, und einer Desinfektionsmittel-Zuführleitung (13) die an den Flüssigkeitskreis (2) angeschlossen und wahlweise entsperrbar ist, und
einem in die Blutbehandlungsmaschine integrierten SOS-Generator (9), welcher durch die Desinfektionsmittel-Zuführleitung (13) mit dem Flüssigkeitskreis (2) fluidverbindbar ist und dafür angepasst ist, eine superoxidierte Lösung oder SOS zur Desinfektion des Flüssigkeitskreises (2) aus Dialysekonzentrat und osmotischem Wasser bereitzustellen
**dadurch gekennzeichnet, dass**
die extrakorporale Blutbehandlungsmaschine dazu angepasst ist, ein Ablassen und Ausspülen von SOS zu verhindern, wenn eine nachfolgende Stillstandzeit ermittelt wurde und die SOS vor der erneuten Inbetriebnahme der extrakorporalen Blutbehandlungsmaschine (1) abzulassen und auszuspülen, und/oder
der SOS-Generator (9) dazu angepasst ist, den Flüssigkeitskreis (2) sowie vorzugsweise an den Flüssigkeitskreis (2) angeschlossene Leitungen unmittelbar vor Stillstandzeiten des Blutbehandlungssystems automatisch zu füllen, wobei ein Desinfektionsprozess unmittelbar vor einer erneuten Inbetriebnahme ausgeführt wird.

2. Extrakorporale Blutbehandlungsmaschine nach Anspruch 1, wobei
der Flüssigkeitskreis (2) einen Wasseraufbereitungsabschnitt (14), vorzugsweise einen Umkehrosmose-Abschnitt, aufweist; und
eine Wasser-Abführleitung (17), durch welche der SOS-Generator (9) wahlweise stromabwärts des Wasseraufbereitungsabschnitts (14) an den Flüssigkeitskreis (2) anschließbar ist.

3. Extrakorporale Blutbehandlungsmaschine nach Anspruch 2, wobei
die Wasser-Abführleitung (17) dazu angepasst ist, einen Teilstrom des osmotischen Wassers zu entnehmen; und
die vom SOS-Generator (9) erzeugte SOS anschließend in den Flüssigkeitskreis (2) zu leiten.

4. Extrakorporale Blutbehandlungsmaschine nach Anspruch 3, wobei die SOS durch eine Dosierpumpe (18), welche an der Desinfektionsmittel-Zuführleitung (13) bereitgestellt ist, dosiert in den Flüssigkeitskreis (2) geleitet wird.

5. Extrakorporale Blutbehandlungsmaschine nach einem der vorstehenden Ansprüche, wobei ein Sensor (6) stromabwärts des SOS-Generators (9) in dem Flüssigkeitskreis (2) vorgesehen ist, welcher dazu angepasst ist, außerhalb eines Desinfektionsprozesses die Chlorfreiheit zu überwachen und/oder mittels welchem während eines Desinfektionsprozesses die Zielkonzentration der SOS in dem Flüssigkeitskreis (2) steuerbar ist.

6. Extrakorporale Blutbehandlungsmaschine nach einem der vorstehenden Ansprüche, wobei stromaufwärts von empfindlichen Einheiten, vorzugsweise des Wasseraufbereitungsabschnitts (14), eine Schutzeinrichtung (15) zum Schutz dieser Einheiten vor Kontakt mit der SOS vorgesehen ist.

7. Extrakorporale Blutbehandlungsmaschine nach einem der vorstehenden Ansprüche, wobei außenseitig an der extrakorporale Blutbehandlungsmaschine (1) ein mit dem SOS-Generator (9) verbundener Ablassanschluss (12), vorzugsweise ein Hahn, vorgesehen ist, welcher einem Nutzer zugänglich ist und dazu dient, dem Nutzer SOS als Desinfektionsmittel bereitzustellen.

8. Extrakorporale Blutbehandlungsmaschine nach Anspruch 1, wobei der Flüssigkeitskreis (2) ein offener Kreis ist, wobei der SOS-Generator (9) an Anschlüsse für Dialysekonzentrat (10) und osmotisches Wasser (11) angeschlossen ist.

9. Verfahren zur Desinfektion eines Flüssigkeitskreises (2), vorzugsweise eines Dialyseflüssigkeitskreises, einer extrakorporalen Blutbehandlungsmaschine (1), vorzugsweise einer Dialysemaschine, nach einem der vorstehenden Ansprüche, mit folgenden Schritten:
- Zuführen von Dialysekonzentrat und osmotischem Wasser zu einem in die extrakorporale Blutbehandlungsmaschine (1) integrierten SOS-Generator (9);
- Erzeugen von superoxidierter Lösung oder SOS durch den SOS-Generator (9);
- Befüllen des Flüssigkeitskreises (2) mit der SOS;
- Einwirken der SOS während einer vorermittelten Desinfektionsdauer; und
- Ablassen sowie Ausspülen der SOS nach Ablauf der Desinfektionsdauer bis eine Konzentration der im Flüssigkeitskreis (2) vorhandenen (Rest-) SOS einen Konzentrationsgrenzwert unterschreitet
**gekennzeichnet durch** folgende Schritte:
- Ermitteln einer Stillstandzeit der Blutbehandlungseinrichtung; und
- Füllen des Flüssigkeitskreises (2) sowie vorzugsweise von an den Flüssigkeitskreis (2) angeschlossenen Leitungen mit SOS unmittelbar vor der Stillstandzeit und Ausführen eines Desinfektionsprozesses vor der erneuten Inbetriebnahme der extrakorporalen Blutbehandlungsmaschine(1); oder
- Verhindern des Ablassens und Ausspülens von SOS wenn eine nachfolgende Stillstandzeit ermittelt wurde und Ablassen und Ausspülen der SOS vor der erneuten Inbetriebnahme der extrakorporalen Blutbehandlungsmaschine (1).

10. Verfahren nach Anspruch 9, ferner mit folgendem Schritt:
- Anschließen des SOS-Generators (9) an den Flüssigkeitskreis (2) über eine Wasser-Abführleitung (17), welche an einen Wasseraufbereitungsabschnitt (14) angeschlossen ist, der in den nunmehr geschlossenen Flüssigkeitskreis (2) zwischengeschaltet ist.

11. Verfahren nach Anspruch 10, ferner mit folgenden Schritten:
- Abführen eines Stroms von osmotischem Wasser, vorzugsweise eines Teilstroms, aus dem Flüssigkeitskreis (2) durch die Wasser-Abführleitung (17) und Zuführen des Teilstroms an den SOS-Generator (9); und
- Zuführen der SOS von dem SOS-Generator (9) in den Flüssigkeitskreis (2), vorzugsweise über eine Dosierpumpe (18).

12. Verfahren nach einem der Ansprüche 9 bis 11, ferner mit folgendem Schritt:
- Steuerung einer Zielkonzentration von SOS in dem Flüssigkeitskreis (2) mit Hilfe eines Sensors (6) und vorzugsweise einer Dosierpumpe (18).

13. Verfahren nach einem der Ansprüche 9 bis 12, ferner mit folgenden Schritten:
- Erkennen von SOS durch einen Sensor einer Schutzeinrichtung (15), welche vorzugsweise stromaufwärts unmittelbar neben empfindlichen Einheiten, vorzugsweise einem Wasseraufbereitungsabschnitt (14), in dem Flüssigkeitskreis (2) angeordnet ist; und;
- Aktivieren der Schutzeinrichtung (15), welche ein Weiterleiten der SOS zu den empfindlichen Einheiten verhindert, insbesondere ein Ablassen der SOS initiiert.

## Claims

1. An extracorporeal blood treatment machine, preferably a dialysis machine, comprising
a fluid circuit (2), preferably a dialysis fluid circuit, and a disinfectant supply line (13) which is connected to the fluid circuit (2) and is selectively releasable, and
an SOS generator (9) integrated in the blood treatment machine which can be fluid-communicated with the fluid circuit (2) through the disinfectant supply line (13) and is adapted for providing a super-oxidized solution or SOS from dialysis concentrate and osmotic water for disinfection of the fluid circuit (2),
**characterized in that**
the extracorporeal blood treatment machine is adapted to prevent draining and flushing of SOS when a subsequent idle time has been determined, and to drain and flush the SOS before starting the operation of the extracorporeal blood treatment machine (1) again, or
the SOS generator (9) is adapted to automatically fill the fluid circuit (2) as well as preferably lines connected to the blood treatment system immediately before idle times of the blood treatment system, with a disinfection process being carried out immediately before starting operation again.

2. The extracorporeal blood treatment machine according to claim 1, wherein
the fluid circuit (2) has a water processing portion (14); and
a water drain line (17) through which the SOS generator (9) can be selectively connected to the fluid circuit (2) downstream of the water processing portion (14).

3. The extracorporeal blood treatment machine according to claim 2, wherein
the water drain line (17) is adapted to remove a partial flow of the osmotic water; and
to subsequently guide the SOS generated by the SOS generator (9) into the fluid circuit (2).

4. The extracorporeal blood treatment machine according to claim 3, wherein the SOS is guided into the fluid circuit (2) after being metered through a metering pump (18) provided at the disinfectant supply line (13).

5. The extracorporeal blood treatment machine according to claim 1, wherein a sensor (6) is provided downstream of the SOS generator (9) in the fluid circuit (2), wherein the sensor (6) is adapted to monitor the freedom of chlorine outside a disinfection process and/or by means of which sensor (6) the target concentration of the SOS in the fluid circuit (2) is controllable during a disinfection process.

6. The extracorporeal blood treatment machine according to claim 1, wherein upstream of sensitive units, preferably the water processing portion (14), a protective unit (15) for protecting said units against contact with the SOS is provided.

7. The extracorporeal blood treatment machine according to claim 1, wherein on the outside of the extracorporeal blood treatment machine (1), a discharge port (12) connected to the SOS generator (9), preferably a spigot, is provided which is accessible to a user and serves for providing the user with SOS as disinfectant.

8. The extracorporeal blood treatment machine according to claim 1, wherein the fluid circuit (2) is an open circuit, with the SOS generator (9) being connected to ports for dialysis concentrate and osmotic water.

9. A method for disinfection of a fluid circuit (2) of an extracorporeal blood treatment machine according to any one of the preceding claims, comprising the following steps of:
- supplying dialysis concentrate and osmotic water to an SOS generator (9) integrated in the extracorporeal blood treatment machine (1);
- generating super-oxidized solution or SOS by the SOS generator (9);
- filling the fluid circuit (2) with the SOS;
- acting of the SOS during a pre-established disinfection period; and
- draining and flushing the SOS after expiry of the disinfection period until a concentration of the (residual) SOS present in the fluid circuit (2) falls below a concentration limit,
**characterized by** the method further comprising the following steps of
- establishing an idle time of the blood treatment apparatus; and
- filling the fluid circuit (2) as well as preferably the lines connected to the fluid circuit (2) with SOS immediately before the idle time and carrying out a disinfection process before the operation of the extracorporeal blood treatment machine is started again; or
- preventing SOS from being drained and flushed, when a subsequent idle time has been established, and draining and flushing of the SOS before operation of the extracorporeal blood treatment machine is started again.

10. The method according to claim 9, further comprising the following step of:
- connecting the SOS generator (9) to the fluid circuit (2) via a water drain line (17) which is connected to a water processing portion (14) being interconnected in the now closed fluid circuit (2).

11. The method according to claim 10, further comprising the following steps of:
- draining a flow of osmotic water, preferably a partial flow, from the fluid circuit (2) through the water drain line (17) and supplying the partial flow to the SOS generator (9); and
- supplying the SOS from the SOS generator (9) into the fluid circuit (2).

12. The method according to one of claims 9 to 11, further comprising the following step of:
- controlling a target concentration of SOS in the fluid circuit (2) with the aid of a sensor (6) and preferably a metering pump (18).

13. The method according to one of claims 9 to 12, further comprising the following steps of:
- identifying SOS by a sensor of a protective unit (15) which is arranged in the fluid circuit (2), preferably upstream directly next to sensitive units, preferably a water processing portion (14); and
- activating the protective unit (15) which prevents the SOS from being transferred to the sensitive units and in particular initiates draining of the SOS.

## Revendications

1. Machine de traitement extracorporel du sang, de préférence machine de dialyse, avec
un circuit de liquide (2), de préférence un circuit de liquide de dialyse, et une conduite d'alimentation en désinfectant (13) qui est raccordée au circuit de liquide (2) et peut être désaccouplée sélectivement de celui-ci, et
un générateur de SOS (9) intégré dans la machine de traitement du sang qui peut être relié de manière fluidique au circuit de liquide (2) par le biais de la conduite d'alimentation en désinfectant (13) et est adapté pour mettre à disposition une solution suroxydée ou SOS destinée à la désinfection du circuit de liquide (2) composée de concentré de dialyse ou d'eau osmosée
**caractérisée en ce que**
la machine de traitement extracorporel du sang est adaptée pour empêcher une vidange et un rinçage de la SOS lorsqu'un temps de repos ultérieur a été déterminé et pour vidanger et rincer la SOS avant la remise en service de la machine de traitement extracorporel du sang (1), et/ou
le générateur de SOS (9) est adapté pour remplir automatiquement le circuit de liquide (2) ainsi que de préférence des conduites raccordées au circuit de liquide (2) directement avant des temps de repos du système de traitement du sang, dans laquelle le processus de désinfection est réalisé directement avant la remise en service.

2. Machine de traitement extracorporel du sang selon la revendication 1, dans laquelle
le circuit de liquide (2) présente une section de traitement de l'eau (14), de préférence une section d'osmose inverse ; et
une conduite d'évacuation de l'eau (17) à travers laquelle le générateur de SOS (9) peut être raccordé au circuit de liquide (2) sélectivement en aval de la section de traitement de l'eau (14).

3. Machine de traitement extracorporel du sang selon la revendication 2, dans laquelle
la conduite d'évacuation de l'eau (17) est adaptée pour retirer un flux partiel de l'eau osmosée ; et
pour conduire ensuite la SOS générée par le générateur de SOS (9) dans le circuit de liquide (2).

4. Machine de traitement extracorporel du sang selon la revendication 3, dans laquelle la SOS est dosée par le biais d'une pompe de dosage (18), qui est mise à disposition au niveau de la conduite d'alimentation en désinfectant (13), et conduite dans le circuit de liquide (2).

5. Machine de traitement extracorporel du sang selon l'une quelconque des revendications précédentes, dans laquelle un capteur (6) est prévu dans le circuit de liquide (2) en aval du générateur de SOS (9), lequel circuit de liquide est adapté pour surveiller l'absence de chlore en dehors d'un processus de désinfection et/ou au moyen duquel la concentration cible de la SOS dans le circuit de liquide (2) peut être commandée pendant un processus de désinfection.

6. Machine de traitement extracorporel du sang selon l'une quelconque des revendications précédentes, dans laquelle en amont d'unité sensibles, de préférence de la section de traitement de l'eau (14), un équipement de protection (15) destiné à la protection de ces unités contre un contact avec la SOS est prévu.

7. Machine de traitement extracorporel du sang selon l'une quelconque des revendications précédentes, dans laquelle à l'extérieur de la machine de traitement extracorporel du sang (1) un raccord de vidange (12) relié au générateur de SOS (9), de préférence un robinet, est prévu, lequel est accessible à un utilisateur et sert à mettre à disposition de l'utilisateur une SOS en tant que désinfectant.

8. Machine de traitement extracorporel du sang selon la revendication 1, dans laquelle le circuit de fluide (2) est un circuit ouvert, dans laquelle le générateur de SOS (9) est raccordée à des raccordements pour le concentré de dialyse (10) et l'eau osmosée (11).

9. Procédé destiné à la désinfection d'un circuit de liquide (2), de préférene d'un circuit de liquide de dialyse, d'une machine de traitement extracorporel du sang (1), de préférene d'une machine de dialyse, selon l'une quelconque des revendications précédentes, avec les étapes suivantes :
- l'alimentation d'un générateur de SOS (9) intégré dans la machine de traitement extracorporel du sang (1) en concentré de dialyse et en eau osmosée ;
- la génération d'une solution suroxydée ou SOS par le biais du générateur de SOS (9) ;
- le remplissage du circuit de liquide (2) avec la SOS ;
- l'action de la SOS pendant une durée de désinfection pré-établie ; et
- la vidange ainsi que le rinçage de la SOS après l'écoulement de la durée de désinfection jusqu'à ce qu'une concentration de la SOS (résiduelle) présente dans le circuit de liquide (2) soit inférieure à une valeur limite de concentration
**caractérisé par**les étapes suivantes :
- la détermination d'un temps de repos de l'équipement de traitement du sang ; et
- le remplissage du circuit de liquide (2) ainsi que de préférence par des conduites raccordées au circuit de liquide (2) avec la SOS directement avant le temps de repos et la réalisation d'un processus de désinfection avant la remise en service de la machine de traitement extracorporel du sang (1) ;
- le fait d'empêcher la vidange et le rinçage de la SOS lorsqu'un temps de repos ultérieur a été déterminé et la vidange et le rinçage de la SOS avant la remise en service de la machine de traitement extracorporel du sang (1).

10. Procédé selon la revendication 9, avec en outre l'étape suivante :
- le raccordement du générateur de SOS (9) au circuit de liquide (2) via une conduite d'évacuation de l'eau (17) qui est raccordée à une section de traitement de l'eau (14) qui est intercalée dans le circuit de liquide (2) désormais raccordé.

11. Procédé selon la revendication 10, avec en outre les étapes suivantes :
- l'évacuation d'un flux d'eau osmosée, de préférence d'un flux partiel, du circuit de liquide (2) par le biais de la conduite d'évacuation de l'eau (17) et l'alimentation du générateur de SOS (9) en flux partiel ; et
- l'alimentation du circuit de liquide (2) avec la SOS par le générateur de SOS (9), de préférence via une pompe de dosage (18).

12. Procédé selon l'une quelconque des revendications 9 à 11, avec en outre l'étape suivante :
- la commande d'une concentration cible de SOS dans le circuit de liquide (2) à l'aide d'un capteur (6) et de préférence d'une pompe de dosage (18).

13. Procédé selon l'une quelconque des revendications 9 à 12, avec en outre les étapes suivantes :
- la reconnaissance de la SOS par le biais d'un capteur d'un équipement de protection (15), qui est disposé dans le circuit de liquide (2) de préférence en amont directement à côté d'unités sensibles, de préférence d'une section de traitement de l'eau (14) ; et ;
- l'activation de l'équipement de protection (15) qui empêche une transmission de la SOS aux unités sensibles, initie en particulier une vidange de la SOS.
